# EUROPEAN PATENT SPECIFICATION

(11) **EP 0 435 837 B1**
(45) Date of publication and mention of the grant of the patent: **15.01.1997**
(21) Application number: 90850370.9
(22) Date of filing: 12.11.1990
(51) Int. Cl.: A61B 6/00, A61B 6/10

(54) **Method and device for controlling the operations of a mammographic x-ray apparatus**
Verfahren und Vorrichtung zur Regelung der Bewegung, Verschiebung eines Röntgenmammographiegeräts
Procédé et dispositif de contrôle des opérations d'un mammographe

(30) Priority: 23.11.1989 FI 895611
(43) Date of publication of application: 03.07.1991
(73) Proprietor: PLANMED OY, 00810 Helsinki (FI)
(72) Inventor: Strömmer, Pekka, SF-02210 Espoo (FI)
(74) Representative: Onn, Thorsten

(56) References cited:
- EP-A- 0 145 893
- WO-A-87/01555
- WO-A-89/11248
- DE-A- 3 227 258

## Description

The invention concerns a method for controlling the operations of a mammographic X-ray apparatus, wherein a breast to be photographed is pressed between a press plate and a film cassette or an equivalent depicting member by a press mechanism.

Further, the invention concerns a device for controlling the operations of a mammographic X-ray apparatus, including a press plate and a film cassette or equivalent depicting member, and a motor for applying compression to the breast.

In mammographic photographing, in order that the photographing should be successful, it is essential that the object to be photographed is placed in the apparatus so that it is in a correct position and forms a layer of minimum thickness so that the amount of secondary radiation scattered from the breast tissue can be minimized. In order that the pressing could be carried out as well as possible while, nevertheless, keeping the discomfort caused to the patient as little as possible, in prior art mammographic apparatuses have been provided with monitor means for the compressed thickness and compression force of the object to be photographed, the function of said means being to aid the nurse when the breast is being pressed. In some prior-art apparatuses, an excessively high compression force additionally stops the compression movement or releases the press mechanism, thereby preventing an inadvertent excessively intensive compression.

In respect of the prior art related to the present invention, reference is made to EP-A-370 089 and EP application No. 93 102 302, which fall under Art. 54(3)EPC.

The object of the present invention is to provide a novel method and device for controlling the operations of a mammographic X-ray apparatus so that the operation of the apparatus is more flexible, safer, and more agreeable to the patient to be photographed.

It is a further object of the invention to provide a device that facilitates correct positioning of the breast both for the nurse and for the patient.

The control method and device in accordance with the present invention are particularly well suitable for a mammographic apparatus similar to that described in the above EP application No. 93 102 302, wherein a particularly advantageous mode and arrangement of equipment are used for pressing a breast in connection with photographing between the holders. An object of the present invention is to provide certain advantages of synergism with said EP patent application. On the other hand, it should be emphasized that the present invention is also suitable for use in a great variety of other mammographic X-ray apparatuses which are known in prior art or which may be developed later.

In view of achieving the objectives stated above and those that will come out later, the method in accordance with the invention is mainly characterized by the steps of: measuring a compression force F applied by a holder means to the breast to be photographed; and on the basis of said measurement, regulating the velocity V of compression movement of the press mechanism by lowering the velocity V starting from a certain force F0 in a linear way to zero until a maximal compression force F1 is reached.

On the other hand, the device in accordance with the invention is mainly characterized in that the device comprises: a detector for measuring the compression force applied to the breast; and a control unit responsive to said quantity to lower velocity of the compression-applying motor starting from a certain force F0 in a linear way to zero until a maximal compression force F1 is reached.

In EP-A-145 893 disclosing a compression system for an X-ray diagnostic apparatus two compression velocities are used. At first the breast is compressed at a rather high velocity. This high velocity is then changed to a lower velocity, which is maintained constant. At the end of the compression the compression member is stopped abruptly. Such stepped changes of velocity cause high inertial forces and vibrations in the compression members. Such vibrations are also felt to be unpleasant to the patient.

DE-A- 3 227 258 discloses a remote-controlled compression tubus for an X-ray apparatus. Said apparatus includes a compression force limiter. The compression velocity can be changed from a lower velocity to a higher one and vice versa. Also when using this apparatus there are abrupt stops entailing the disadvantages mentioned above.

In the invention, it has been realized, in a novel way, to utilize the breast compression force, which has been measured in a way known in prior art, besides for monitoring and for switching-off the compression, also for control of the apparatus in a considerably more versatile way than in prior art.

In the solution in accordance with the present invention, on the basis of the measurement of compression force, the velocity of the compression movement of the press mechanism, the velocity of the up/down movement of the apparatus, the movement of rotation of the stand, and/or the X-ray generator, or only some of them, are regulated. The invention is primarily intended for use in a mammographic apparatus in which said movements are motorized, so that the mechanical actuators can be integrated in the control system in a novel way.

In the following, the invention will be described in detail with reference to some exemplifying embodiments of the invention illustrated in the figures in the accompanying drawing, the invention being not confined to the details of said embodiments.
Figure 1 is a schematic side view of the construction of the apparatus.
Figure 2 shows the measurement and control system as a block diagram.
Figure 3 illustrates the velocity of the press motor as a function of the compression force.
Figure 4 illustrates the press velocity as a function of the compression force in a preferred special case.

To begin with, the basic construction of the mammographic apparatus shown in Fig. I will be described. The photographing stand consists of a C-arm 1, at one of whose ends the X-ray tube 6 is fitted, whereas the film cassette 5 is placed at the other end. The C-arm 1 can be rotated around the shaft 8 in relation to the frame 10 of the apparatus by means of a motor 9. For photography, the breast 4 to be photographed is pressed between the film cassette 5 and the press plate 2 by means of the press motor 7, at the same time as the compression force is measured by means of a detector 3. The height of the C-arm 1 is regulated by means of a motor 11.

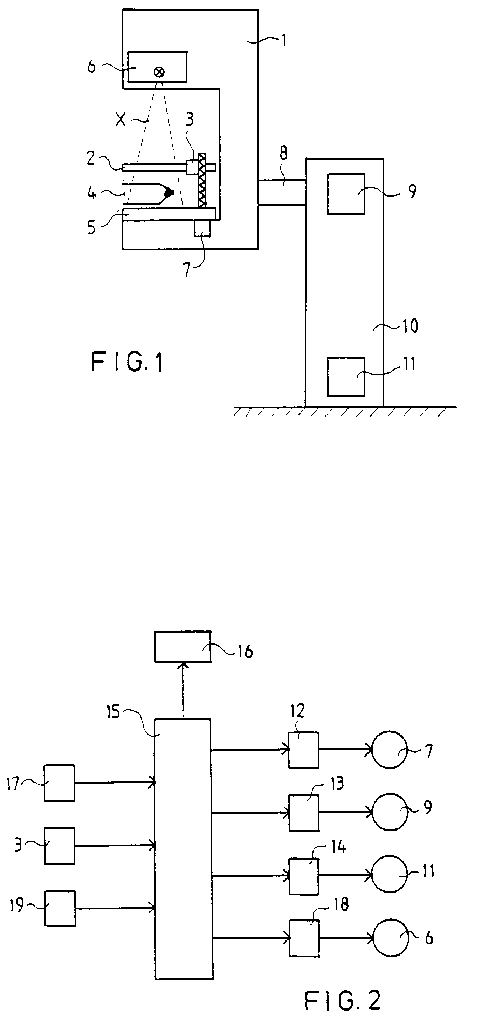

In respect of further details of construction of the X-ray apparatus shown schematically in Fig. 1, reference is made to EP-A-370 089 and EP application No. 93 102 302 mentioned above.

Fig. 2 shows the construction of the control system as a block diagram. The control unit 15 receives the control data from a set member 17, which may be, for example, a keyboard. Also, the control unit 15 monitors the operation of the stand by means of limit detectors 19 and a compression-force measurement detector 3. On the basis of these data, the control unit 15 sets the data of the monitor 16 and controls the press motor 7 by the intermediate of the controller 12, the rotation motor 9 by the intermediate of the controller 13, and the lifting motor 11 by the intermediate of the controller 14. Also, the control unit 15 controls the generator 18 and further, by its means, the X-ray tube 6. The detailed construction of the control unit 15 itself will not be discussed in more detail in this connection, because its embodiment has no relevance to the present invention. For example, the control unit may be a microprocessor with necessary auxiliary equipment.

An object of the invention is to facilitate the photographing operation and to prevent damage in a situation in which the breast 4 is already partly pressed between the film cassette 5 and the press plate. In the solution in accordance with the present invention, compression measurement is used in accordance with the diagram shown in Fig. 2 for controlling the operation of the apparatus in accordance with the following description.

As a rule, the process of photography proper proceeds so that first the desired projection of photography is chosen, which determines the position of rotation of the C-arm 1, of which positions the five most typical ones are vertical position, horizontal positions rotated to either directions and positions inclined to either direction by about 45 to 60 degrees. Thereupon the holder stand is placed at a level in accordance with the height of the person to be photographed and the breast 4 is placed between the press plate 2 and the film cassette 5 and pressed in its place, whereupon the photographing is carried out by means of an X-ray beam X.

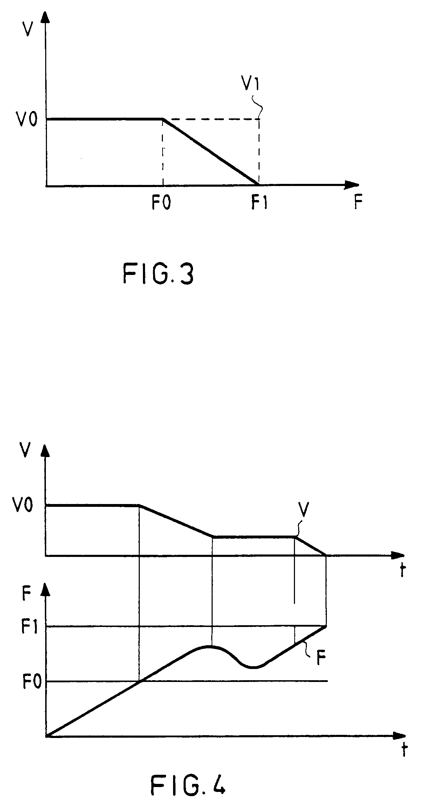

When the breast 4 is compressed by means of the press plate 2, in the invention the velocity of the press motor 7 is made slower as the compression force is increasing until, after the maximum permitted compression force has been reached, the press motor 7 stops completely and prevents excessive compression forces. in Fig. 3 this is shown as a graph, wherein the vertical axis V represents the velocity of the press motor 7 and the horizontal axis F represents the compression force. Initially, the press motor 7 is run at a relatively high invariable velocity V0, but when the measured compression force reaches a predetermined limit F0, the velocity V starts being slowed down until, after the force F has reached a predetermined limit F1, the velocity V has been regulated to zero.

The dependence of the press velocity V on the measured force F may be linear or non-linear. A high initial velocity V0 permits pre-compression of the breast 4 quickly, which is desirable because this stage is difficult and requires holding of the breast 4 in its place by hand until the press plate 2 starts positioning the breast 4 stably. After a reasonable compression has been reached. the velocity V starts slowing down, whereby a careful and accurate compression is accomplished and there is no risk of unnecessarily high compression force as a result of an excessively high velocity.

During slow pressing, the correct compression strength of the breast 4 can be measured accurately, whereby it is possible to employ just a compression force as high as is really necessary to take a good photograph. It is a well-known fact that the operation is usually disagreeable for the patient, and therefore the compression strength should be kept at such a level that at least it does not cause actual pain to the patient to be photographed.

In Fig. 3, the dashed line V1 illustrates the compression velocity of the prior-art solutions, which remains invariable until the present limit F₁ of compression force is reached and the press movement is suddenly stopped. In this method, the breast is usually compressed up to the limit, whereupon the pressing is continued manually by means of a mechanism separately provided in the apparatus for this purpose. Such a solution is operable but mechanically difficult to carry into effect and time-consuming. Moreover, the limit should be set individually for each patient based on sensing of the properties of the breast, but, as a rule, the limit remains fixed at a value sufficiently high for all cases.

Fig. 4 illustrates a special application of the invention. To start with, the compression velocity is invariable V0 and, when the force F reaches a predetermined level F0, the compression velocity V starts being slowed down in the way described above. However, if the compression force F is lowered during further compressing, the velocity V is not increased accordingly, but the velocity is kept at the value to which it has been slowed down. When the force F becomes higher further, the velocity is slowed down in the way described above until, when the force F reaches a predetermined limit F1, the velocity has been lowered to zero. A situation of this sort may arise, for example, when a press plate described in EP application No. 93 102 302 is used, in which case an increase in the velocity in between may seem scaring, and in this way a disagreeable situation is eliminated.

Occasionally, the compression must also be reduced during positioning of the breast 4. In such a case, the releasing can be carried out either rapidly at full velocity or slowly by increasing the velocity in relation to the compression force. It is known in prior art to open the compression quickly, but in such a case the compression may be opened excessively and the breast no longer remains in the desired position, in which case the positioning must be restarted from the beginning. In the solution in accordance with the invention, the opening of the compression can be accomplished optionally either quickly or in relation to the compression force, whereby said drawbacks are avoided.

When the breast 4 is partly compressed, occasionally there is still a need to adjust the height of the C-arm 1. In the solution in accordance with the present invention, a raising movement is in such a case permitted by the control system, but only at a very slow velocity if the compression measurement ascertains that there is a compression. As a rule, in prior-art mammographic apparatuses, the raising movement is carried out manually, in which case a height adjustment with the patient partly compressed is impossible or very difficult, and if it is carried out by means of a motor, careless operation of the apparatus may result in a height adjustment at an excessively high velocity, which is quite a scaring experience for the patient fixed to the C-arm 1. In the solution in accordance with the invention, adjustment of the height of the C-arm 1 is easy and, at the same time, more agreeable to the patient.

The movement of rotation of the C-arm 1 around the shaft 8 is, as a rule, carried out manually, but motor-operated solutions also exist. In the solution in accordance with the invention, the control of the compression measurement is also employed for controlling the movement of rotation so that it prevents movement of rotation as long as compression can be noticed. In motorized models, the prevention can be accomplished by preventing control of the motor, and in manual models by locking the movement, for example, by means of a solenoid. As an exceptional case, in motorized models, it is possible to permit a rather short slow rotation, for example, necessary in stereo photography, wherein the breast holders are not permanently fixed to the movement of rotation of the C-arm but, nevertheless, the patient supports herself on the C-arm 1. A high-speed rotation is not desirable when the patient is under compression.

A novel feature, which may be combined with the invention when necessary, is the use of compression measurement also in the control of the X-ray generator 6. X-ray radiation can be prevented if the compression measurement does not find a sufficiently high compression force. In such a case, inadvertent irradiation or photography of a deficiently positioned breast is prevented, which is a substantial advantage.

In the following, the patent claims will be given, and the various details of the invention may show variation within the scope of the inventive idea defined in said claims.

## Claims

1. Method for controlling the operations of a mammographic X-ray apparatus, wherein a breast (4) to be photographed is pressed between a press plate (2) and a film cassette (5) or an equivalent depicting member by a press mechanism, **characterized** by the steps of:
measuring a compression force (F) applied by a holder means to the breast (4) to be photographed, and
on the basis of said measurement, regulating the velocity (V) of compression movement of the press mechanism by lowering the velocity (V) starting from a certain force (F0) in a linear way to zero until a maximal compression force (F1) is reached.

2. Method as in claim 1, wherein the breast holder means is controlled to a compression position initially at a substantially invariable velocity (V0) until the compression force reaches a certain preset value (F0), whereupon the compression velocity (V), controlled by the force measurement, is lowered until a maximal compression force (F1) is reached, by which time the velocity has been adjusted to zero.

3. Method as in any of claims 1 and 2, wherein the compression on the breast (4) is released during positioning of the breast, and that the releasing of the compression is carried out either at full velocity or at partial velocity while correspondingly increasing the velocity as the compression force becomes lower.

4. Method as in any of claims 1-3, wherein a control responsive to measurement of compression force (F) is used in the control of movement of a C-arm (1) or equivalent of the mammographic apparatus so that a movement of rotation of the C-arm is substantially prevented when a compression force is detected.

5. Method as in any of claims 1-4, wherein by means of measurement of the compression force of the breast holder means, an X-ray generator (6) associated with the apparatus is controlled so that X-ray radiation is prevented at compression forces lower than a certain value.

6. Method as in claim 2 wherein
on the basis of said measurement, the velocity (V) of compression movement of the press mechanism is regulated so that, after a certain compression movement of invariable velocity (V0), the compression velocity is lowered until the compression force reaches the certain present value (F0), whereupon the compression is continued with a lower substantially invariable velocity; and
the final stage of compression is carried out while slowing the velocity (V) towards zero until the preset maximal compression force (F1) is reached.

7. Device for controlling the operation of a mammographic X-ray apparatus including a press plate (2) and a film cassette (5) or equivalent depicting member, and a motor (7) for applying compression to the breast, **characterized** by
a detector (3) for measuring the compression force applied to the breast; and
a control unit (15) responsive to said quantity to lower velocity of the compression-applying motor (7) starting from a certain force (F0) in a linear way to zero until a maximal compression force (F1) is reached.

## Patentansprüche

1. Verfahren zur Regelung der Arbeitsweise eines Röntgenmammographiegeräts, wobei eine zu fotographierende Brust (4) zwischen einer Anpressplatte (2) und einer Filmkassette (5) oder einem gleichwertigen Aufzeichnungsmittel durch eine Presseinrichtung zusammengepreßt wird, gekennzeichnet durch folgende Schritte:
Messen einer durch eine Halteeinrichtung an die zu fotographierende Brust (4) aufgebrachte Druckkraft (F) und
auf Basis dieser Messung, ein Reguiieren der Geschwindigkeit (V) der Pressbewegung der Presseinrichtung durch Vermindern der Geschwindigkeit (V), beginnend bei einem bestimmten voreingestellten Wert (FO), und zwar in linearer Form bis auf 0, bis eine maximale Druckkraft (F1) erreicht wird.

2. Verfahren nach Anspruch 1,
wobei die Brusthalteeinrichtung bis zu einem Druckzustand gesteuert wird, und zwar anfangs mit einer im wesentlichen konstanten Geschwindigkeit (VO) bis die Druckkraft einen bestimmten festgelegten Wert (FO) erreicht, worauf die Pressgeschwindigkeit (V), gesteuert durch die Kraftmessung, vermindert wird, bis eine maximale Druckkraft (F1) erreicht wird, wobei gleichzeitig die Geschwindigkeit auf 0 eingestellt wurde.

3. Verfahren nach einem der Ansprüche 1 oder 2,
wobei der Druck auf die Brust (4) während der Positionierung der Brust weggenommen wird und wobei das Wegnehmen des Druckes entweder bei voller Geschwindigkeit oder bei einer Teilgeschwindigkeit geschieht, während die Geschwindigkeit entsprechend zunimmt; in dem Maße, wie die Druckkraft abnimmt.

4. Verfahren nach einem der Ansprüche 1 bis 3,
wobei eine Überwachungseinrichtung zur Messung der Druckkraft (F) bei der Steuerung der Bewegung eines C-Bügels (1) (bzw. C-Rahmens) oder etwas gleichwertigem des Mammographiegeräts verwendet wird, und zwar so, daß eine Drehbewegung des C-Bügels im wesentlichen ausgeschlossen ist, wenn eine Druckkraft festgestellt wird.

5. Verfahren nach einem der Ansprüche 1 bis 4,
wobei mittels einer Messung der Druckkraft der Brusthalteeinrichtung ein mit dem Gerät verbundener Röntgengenerator (6) so gesteuert wird, daß Röntgenstrahlung bei Druckkräften, die unter einem bestimmten Wert liegen, verhindert wird.

6. Verfahren nach Anspruch 2,
wobei auf Basis dieser Messung die Geschwindigkeit (V) der Pressbewegung der Presseinrichtung so geregelt wird, daß nach einer bestimmten Pressbewegung mit konstanter Geschwindigkeit (VO) die Pressgeschwindigkeit abnimmt, bis die Druckkraft den bestimmten voreingestellten Wert (FO) erreicht, wobei das Pressen mit einer niedrigeren, im wesentlichen konstanten Geschwindigkeit weiterverläuft; und
die Endstufe des Pressens während der Verlangsamung der Geschwindigkeit (V) bis auf 0 ausgeführt wird, und zwar bis die voreingestellte maximale Druckkraft (F1) erreicht wird.

7. Vorrichtung zur Steuerung des Betriebes eines Röntgenmammographiegerätes, die eine Anpressplatte (2) und eine Filmkassette (5) oder ein gleichwertiges Aufzeichnungsmittel, sowie einen Motor (7) zum Ausüben eines Druckes auf die Brust aufweist, **gekennzeichnet durch**
einen Detektor (3) zum Messen der auf die Brust ausgeübten Druckkraft; und
eine Steuereinheit (15), die auf diese Größe reagiert, um die Geschwindigkeit des Motors (7) für die Druckaufbringung herabzusetzen, und zwar ausgehend von einer bestimmten Kraft (FO), in linearer Form abfallend auf 0, bis eine maximale Druckkraft (F1) erreicht ist.

## Revendications

1. Procédé pour le contrôle des opérations d'un appareil de radiomammographie, dans lequel le sein (4) à photographier est comprimé entre une plaque de compression (2) et une cassette de film (5) ou un élément d'imagerie équivalent par un mécanisme de compression, caractérisé par les étapes consistant à :
mesurer une force de compression (F) appliquée sur un moyen de retenue sur le sein (4) à photographier, et
en fonction de cette mesure, réguler la vitesse (V) du mouvement de compression du mécanisme de presse en abaissant la vitesse (V) en partant d'une certaine force (FO) d'une manière linéaire vers zéro jusqu'à l'obtention d'une force de compression maximum (F1).

2. Procédé selon la revendication 1, dans lequel le moyen de retenue du sein est commandé sur une position de compression initialement à une vitesse sensiblement invariable (VO) jusqu'à ce que la force de compression atteigne une certaine valeur préétablie (FO), après quoi la vitesse de compression (V), commandée par la mesure de force, est abaissée jusqu'à l'obtention d'une force de compression maximum (F1), moment auquel la vitesse a été ajustée à zéro.

3. Procédé selon l'une quelconque des revendications 1 et 2, dans lequel la compression sur le sein (4) est libérée pendant le positionnement du sein, et en ce que la libération de la compression s'effectue à pleine vitesse ou à vitesse partielle tout en augmentant de façon correspondante la vitesse à mesure que la force de compression diminue.

4. Procédé selon l'une quelconque des revendications 1-3, dans lequel une commande sensible à la mesure de la force de compression (F) est utilisée dans la commande du mouvement d'un bras en C (1) ou équivalent de l'appareil mammographique de façon à empêcher sensiblement un mouvement de rotation du bras en C lorsqu'une force de compression est détectée.

5. Procédé selon l'une quelconque des revendications 1-4, dans lequel au moyen de la mesure de la force de compression du moyen de retenue du sein, on commande un générateur de rayons X (6) associé à l'appareil de telle sorte que l'on empêche une radiation de rayons X aux forces de compression inférieures à une certaine valeur.

6. Procédé selon la revendication 2, dans lequel
en fonction de cette mesure, on régule la vitesse (V) du mouvement de compression du mécanisme de presse de telle sorte qu'après un certain mouvement de compression de vitesse invariable (VO), la vitesse de compression est diminuée jusqu'à ce que la force de compression atteigne la valeur préétablie (FO), après quoi on poursuit la compression avec une vitesse inférieure sensiblement invariable ; et
l'étape finale de compression s'effectue tout en ralentissant la vitesse (V) en direction de zéro jusqu'à ce que la force de compression maximum préétablie (F1) soit atteinte.

7. Dispositif pour commander le fonctionnement d'un appareil radiomammographique comprenant une plaque de compression (2) et une cassette de film (5) ou élément d'imagerie équivalent, et un moteur (7) pour appliquer une compression au sein, caractérisé par
un détecteur (3) pour mesurer la force de compression appliquée au sein, et
une unité de commande (15) sensible à cette quantité pour réduire la vitesse du moteur (7) appliquant la compression en partant d'une certaine force (FO) d'une manière linéaire en direction de zéro jusqu'à l'obtention d'une force de compression maximum (F1).
